(19) Europäisches Patentamt
European Patent Office
Office européen des brevets

(11) EP 0 584 854 B1

(12) # EUROPEAN PATENT SPECIFICATION

(45) Date of publication and mention of the grant of the patent:
**07.01.1998 Bulletin 1998/02**

(51) Int Cl.[6]: **G01N 33/535**, G01N 33/543, C07D 223/26

(21) Application number: **93202292.4**

(22) Date of filing: **04.08.1993**

(54) **Immunoassay using carbamazepine analogues labelled with horseraddish peroxidase**

Immunoassays unter Verwendung von, mit Meerrettich-Peroxidase markierten Carbamazepin-Analogen

Essai immunologique utilisant des analoques de carbamazepine marqués avec la peroxidase de raifort

(84) Designated Contracting States:
**AT BE CH DE FR GB IT LI NL SE**

(30) Priority: **07.08.1992 US 926202**
**07.08.1992 US 926203**

(43) Date of publication of application:
**02.03.1994 Bulletin 1994/09**

(73) Proprietor: **Johnson & Johnson Clinical Diagnostics, Inc.**
**Rochester New York 14650 (US)**

(72) Inventors:
- **Brummond, Barbara Ann,**
**c/o EASTMAN KODAK COMPANY**
**Rochester, New York 14650-2201 (US)**
- **Saini, Mohan Singh,**
**c/o EASTMAN KODAK COMPANY**
**Rochester, New York 14650-2201 (US)**
- **Ponticello, Ignazio Salvatore,**
**c/o EASTMAN KODAK**
**Rochester, New York 14650-2201 (US)**

(74) Representative: **Mercer, Christopher Paul et al**
**Carpmaels & Ransford**
**43, Bloomsbury Square**
**London WC1A 2RA (GB)**

(56) References cited:

| | |
|---|---|
| **EP-A- 0 304 163** | **EP-A- 0 430 371** |
| **EP-A- 0 468 585** | **EP-A- 0 517 327** |
| **EP-A- 0 517 338** | **DE-A- 2 649 903** |

- **DATABASE WPI Derwent Publications Ltd., London, GB; AN 79-78142B & JP-A-54 119 026 (DAINIPPON) 14 September 1979**
- **PATENT ABSTRACTS OF JAPAN & JP-P-57 109 724 (CHUGAI PHARMA) 8 July 1982**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 584 854 B1

## Description

This invention relates to clinical chemistry, particularly labeled carbamazepine analogues and their use in immunoassays.

Immunoassays, which take advantage of natural immunological reactions, have found wide-spread use as analytical techniques in clinical chemistry. Because of the specificity of the reactions, they are particularly advantageous in quantifying biological analytes that are present in very low concentration in biological fluids. Such analytes (called ligands herein) include, for example, antibodies, therapeutic drugs, narcotics, enzymes, hormones, proteins, and so forth.

In competitive binding immunoassays, a labeled ligand, including immunocompetent derivatives and analogs of the ligand is placed in competition with unlabeled ligand for reaction with a fixed amount of the appropriate binding material (called a receptor herein). Unknown concentrations of the ligand can be determined from the measured signal of either the bound or unbound (i.e. free) labeled ligand. The reaction proceeds as follows:

$$\text{ligand + labeled ligand + receptor} \rightarrow \text{ligand-receptor + labeled ligand-receptor.}$$

Conventional labels include radioactive tags, enzymes, chromophores, fluorophores, stable free radicals, and enzyme cofactors, inhibitors and allosteric effectors.

Consistent with the foregoing an immunoassay for ligands such as carbamazepine in serum can be based on competition between (1) an enzyme labeled carbamazepine analogue with (2) the carbamazepine in a patient's blood serum for immobilized antibody binding sites (see for example JP-A-54 119 026).

Specific requirements for the labeled carbamazepine analogue include: 1) at least 70 - 90% of the analogue can be bound by excess immobilized carbamazepine antibodies; 2) affinity of the analogue for immobilized antibodies is such that competition of a fixed amount of carbamazepine occurs in a therapeutically relevant concentration range; and 3) stability of the analogue against hydrolysis of its enzyme label under storage conditions. Requirements imposed on the carbamazepine analogue include: 1) accessibility of the derivative to the immobilized antibody following conjugation with the enzyme label; 2) specific recognition of the derivative by the antibody to the carbamazepine; 3) sufficient reactivity of the derivative with the enzyme label, either directly or following activation of the enzyme or derivative, under conditions that do not adversely affect enzyme activity; 4) stability of the label against hydrolysis of the carbamazepine analogue from the enzyme; and 5) fast and complete attachment of the carbamazepine hapten to the enzyme by covalent bonding, without denaturing the enzyme.

The present invention provides novel labeled carbamazepine analogues, immunoassay elements and an immunoassay method for carbamazepine.

The labeled carbamazepine analogue has a structure according to claim 1. The method is conducted according to claim 7. The immunoassay element is according to claim 11.

One embodiment of the carbamazepine analogues of claim 1 has the structure according to claim 2.

The labeled carbamazepine analogues, having short linking chains between the carbamazepine nucleus and labels, such as horseradish peroxidase (HRP) are useful with some immobilized antibodies. Analogues with a longer linking group between the Label and the carbamazepine nucleus were strongly bound by the immobilized antibodies tested. Linking groups having amide bonds instead of ester bonds are resistant to hydrolysis and therefore avoid any problem of separation of the carbamazepine nucleus from the label. The carbamazepine-HRP labels of this invention with extended linkers have the ability to be completely bound (>90%) by all the immobilized antibody types we have prepared. This allows us to pursue a variety of antibodies to develop the carbamazepine enzyme immunoassay.

We have devised a new method for the preparation of labeled carbamazepine analogues used in the immunoassay of this invention. The new method of preparation comprises the steps of:

1) contacting

(A) a label having an amine or sulfhydryl group thereon, with an excess of a
(B) carbamazepine analogue comprising:

(i) an active ester group such as succinimidoxycarbonyl;
(ii) a carbamazepine nucleus; and
(iii) a linking chain (i) linking the carboxamide group of the carbamazepine nucleus to the active ester group through a carbonyl group (The linking group is otherwise identical to the linking group defined previously herein); and

2) removing the unused carbamazepine analogue and condensation by-products, preferably by dialysis.

Preferably the step of contacting is carried out by dissolving both the analogue and the label in a water-miscible organic solvent such as N,N-dimethylformamide or dimethyl sulfoxide or a mixture of solvent and water (buffered) before mixing together.

The following examples 1-9 describe the preparation of carbamazepine analogues from which the labeled carbamazepine analogues are prepared.

Preparatory Example 1

N-[2-(3-Succinimidoxycarbonylpropionyloxy)ethyl]carbamazepine

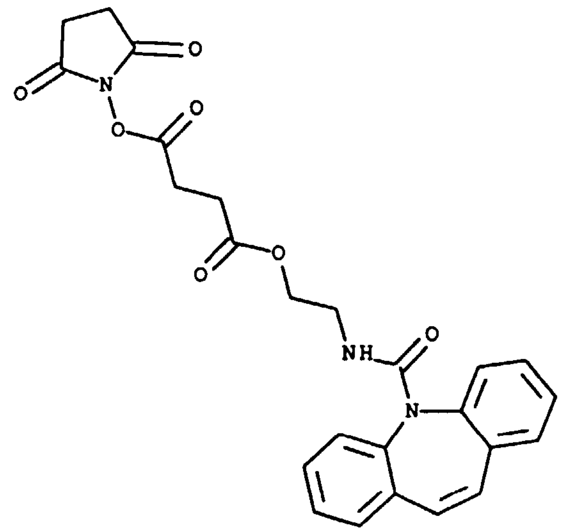

Step 1: Preparation of N-(2-Hydroxyethyl)carbamazepine

A mixture of ethanolamine (6.1 g, 0.1 mole) and 5-chlorocarbonyl-2,2'-iminostilbene (6.5 g, 0.025 mole) in toluene (250 mL) was heated at reflux for 4 hours and then allowed to stand at ambient temperature for 16 hours. To the mixture was added dichloromethane (500 mL), and the solution was washed with 10% hydrochloric acid (2 x 100 mL), washed with saturated sodium bicarbonate solution (100 mL), washed with saturated sodium chloride solution (100 mL), dried over anhydrous magnesium sulfate, filtered, and the solvent removed on a rotary evaporator.

To the residue was added dichloromethane (45 mL) and ethyl acetate (75 ML), and the mixture was placed in the freezer (-16°C). The solid was filtered.

Step 2: N-[2-(3-Carboxypropionyloxy)ethyl]carbamazepine

A mixture of N-(2-hydroxyethyl)carbamazepine (5.6 g, 0.02 mole), succinic anhydride (2.2 g, 0.02 mole), and dimethylaminopyridine (2.4 g, 0.02 mole) in chloroform (25 mL) was stirred at ambient temperature for 24 hours. Dichloromethane (400 mL) was added, and the mixture was washed with 10% hydrochloric acid solution (2 x 100 mL), washed with saturated sodium chloride solution (100 mL), dried over anhydrous magnesium sulfate, filtered, and the solvent removed. To the residue was added ethyl ether (25 mL) and petroleum ether (25 mL). A sample was recrystallized from methanol.

Step 3: N-[2-(3-Succinimidoxycarbonylpropionyloxy)ethyl]carbamazepine

A mixture of N-[2-(3-carboxypropionyloxy)ethyl]carbamazepine (3.8 g, 0.01 mole), N,N'-dicyclohexylcarbodiimide (2.1 g, 0.01 mole), and N-hydroxysuccinimide (1.2 g, 0.01 mole) in chloroform (75 mL) was stirred at room temperature for 20 hours. The mixture was filtered, and the filtrate was concentrated on a rotary evaporator in vacuo to give a white solid. Analytical calculated for $C_{25}H_{23}N_3O_7$: C, 62.89; H, 4.86;N, 8.80. Found C, 60.74: H, 5.12; N, 8.83.

Preparatory Example 2

N-[3-(3-Succinimidoxycarbonylpropionamido)propyl]carbamazepine

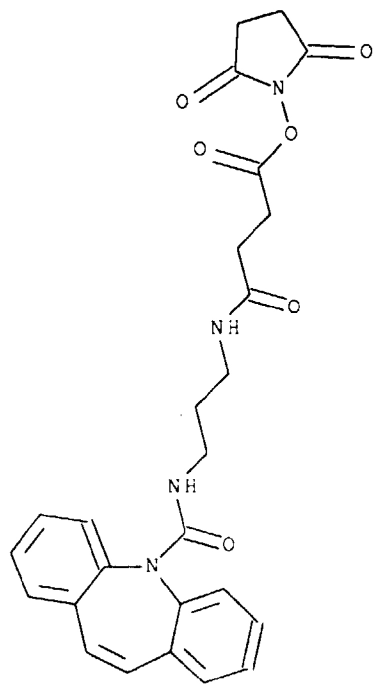

Step 1: N-[3-(Benzyloxycarbonylamino)propyl]carbamazepine

A mixture of N-benzyloxycarbonyl-1,3-propanediamine (8.0 g, 0.04 mole) and triethylamine (5.0 g, 0.05 mole) in chloroform (75 mL) was added dropwise over 15 minutes to 5-chlorocarbonyl-2,2'-iminostilbene (7.6 g, 0.03 mole) in chloroform (200 mL). The mixture was then heated at reflux for 1 hour and at ambient temperature for 16 hours. Dichloromethane (500 mL) was added, and the mixture was washed with 10% hydrochloric acid (2 x 100 mL), washed with saturated sodium chloride solution (100 mL), dried over anhydrous magnesium sulfate, filtered, and the solvent removed on a rotary evaporator in vacuo. To the residue was added ethyl acetate (50 mL) and the solution was placed in the freezer for 2 hours and filtered.

Step 2: N-(3-Aminopropyl)carbamazepine Hydrobromide

N-[3-(Benzyloxycarbonylamino)propyl]carbamazepine (13.2 g, 0.03 mole) and 30-35% hydrogen bromide-acetic acid solution (70 mL) was allowed to stir at room temperature for 1 hour. This mixture was then poured into diethyl ether (3 L), and the solid which forms was tetrated with fresh portions of ether (3 x 1L). The solid was filtered.

Step 3: N-[3-(3-Carboxypropionamido)propyl]carbamazepine

A mixture of N-(3-aminopropyl)carbamazepine hydrobromide (7.5 g, 0.02 mole), triethylamine (2.0 g, 0.02 mole), and succinic anhydride (2.0 g, 0.02 mole) in chloroform (200 mL) was heated for 30 minutes at 50-60°C and allowed to stand at ambient temperature for 20 hours. Dichloromethane (500 mL) was added, and the mixture was washed with 10% hydrochloric acid (2 x 100 mL) and saturated sodium chloride solution (100 mL), then dried over anhydrous magnesium sulfate, filtered, and the solvent removed on a rotary evaporator in vacuo. To the residue was added dichloromethane (100 mL) and petroleum ether (100 mL), and it was placed in the freezer overnight. The solid was filtered.

Step 4: N-[3-(3-Succinimidoxycarbonylpropionamido)propyl]carbamazepine

A mixture of N-[3-(3-carboxypropionamido)propyl]carbamazepine (3.3 g, 0.01 mole), N,N'-dicyclohexylcarbodiimide (2.0 g, 0.01 mole), and N-hydroxysuccinimide (1.0 g, 0.01 mole) in chloroform (80 mL) was stirred at room temperature for 20 hours. The mixture was filtered and the solvent removed on a rotary evaporator in vacuo to give 4.7 g. The solid was dissolved in dichloromethane (20 mL), filtered and the solvent removed. This procedure was repeated an additional time to give 3.0 g (64% yield). Analytical calculated for $C_{26}H_{26}N_3O_6$: C, 65.54; H, 5.50; N, 8.82. Found: C, 62,38; H, 5.47; N, 11.02.

Preparatory Example 3

N-[3-(4-Succinimidoxycarbonylbutyramido)propyl]carbamazepine

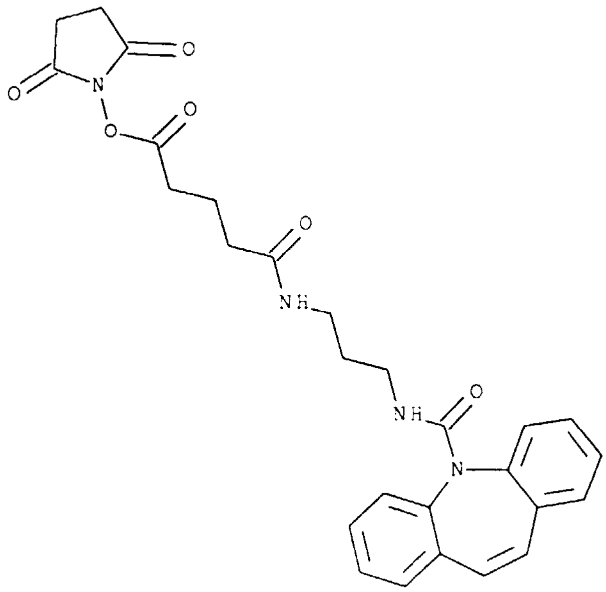

Step 1: N-[3-(4-Carboxybutyramido)propyl]carbamazepine

N-(3-aminopropyl)carbamazepine hydrobromide (4.8 g, 0.0128 mole) was treated with glutaric anhydride (1.5 g, 0.0128 mole), triethylamine (1.4 g, 0.014 mole) by the procedures described in step 3 of Preparatory Example 2.

Step 2: N-[3-(4-Succinimidoxycarbonylbutyramido)propyl]carbamazepine

N-[3-(4-Carboxybutyramido)propyl]carbamazepine was treated with N-hydroxysuccinimide by the procedure described in step 4 of Preparatory Example 2 to give the product. Analytical calculated for $C_{27}H_{28}N_4O_6$: C, 64.28; H, 5.59; N, 11.10. Found: C, 63.84; H, 5.72, N, 10.89.

Preparatory Example 4

N-[6-(4-Succinimidoxycarbonylbutyramido)hexyl]carbamazepine

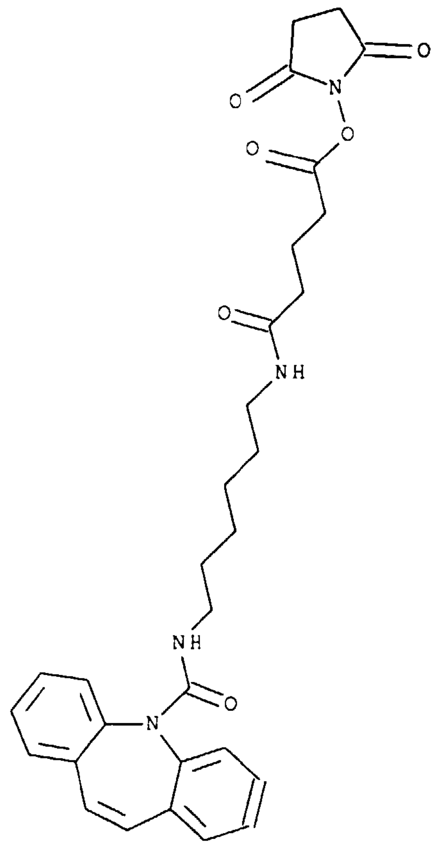

Step 1: N-[6-(Benzyloxycarbonylamino)hexyl]carbamazepine

This material was prepared using the procedure outlined in step 1, Preparatory Example 2, except using N-benzyloxycarbonyl-1,6-hexanediamine in place of the N-benzyloxycarbonyl-1,3-propanediamine, to give 11.0 g (94%, yield). Pure material was obtained by crystallization from ethyl acetate/pentane (1:1).

Step 2: N-(6-Aminohexyl)carbamazepine Hydrobromide

This material was prepared using the procedure outlined in Preparatory Example 2, step 2, except substituting N-[6-(benzyloxycarbonylamino)hexyl]carbamazepine for the N-[3-benzyloxycarbonylamino)propyl]carbamazepine, to give 8.5 g (100% yield).

Step 3: N-[6-(4-Carboxybutyramido)hexyl]carbamazepine

This material was prepared using the procedure outlined in step 3, Preparatory Example 2, except substituting N-(6-aminohexyl)carbamazepine hydrobromide and glutaric anhydride, respectively, for the N-(3-aminopropyl)carbamazepine hydrobromide and succinic anhydride. The product was crystallized from dichloromethane/ethyl acetate (1:1).

Step 4: N-[6-(4-Succinimidoxycarbonylbutyramido)hexyl] carbamazepine

This material was prepared using the procedure outlined in Preparatory Example 2, step 4, except substituting N-[6-(4-carboxybutyramido)hexyl]carbamazepine for the N-[3-(3-carboxypropionamido)propyl]carbamazepine. Analytical calculated for $C_{30}H_{34}N_4O_6$: C, 65,92; H, 6.27; N, 10.25. Found: C, 65.20; H, 6.19; N, 10.02.

Preparatory Example 5 - 5-[4-(4-Succinimidoxycarbonylbutyryl)piperazinocarbonyl]-5H-dibenzo[b,f]azepine

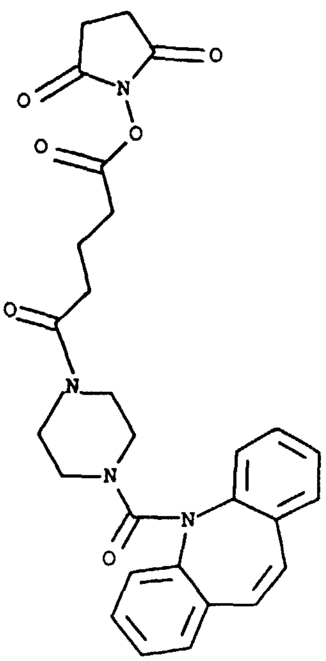

Step 1: 5-[4-(Benzyloxycarbonyl)piperazinocarbonyl]-5H-dibenzo[b,f]azepine

This material was prepared using the procedures outlined in Preporatory Example 2, step 1, except substituting benzyl 1-piperazinecarboxylate in place of the N-benzyloxycarbonyl-1,3-propanediamine.

The compound was dissolved in ethyl ether (10 mL) and petroleum ether was added to the cloud point. The mixture was placed in a freezer, and then filtered to give 9.3 g material.

Step 2A: 5-(Piperazinocarbonyl)-5H-dibenzo[b,f]azepine Hydrobromide

Step 2B: 5-[4-(4-Carboxybutyryl)piperazinocarbonyl]-5H-dibenzo[b,f]azepine

These materials were prepared using the procedures outlined in Steps 2 and 3 of Preparatory Example 2, except starting with 5-[4-(benzyloxycarbonyl)piperazinocarbonyl]-5H-dibenzo[b,f]azepine in place of the N-[3-(benzyloxycarbonylamino)propyl]carbamazepine in step 2, and thus the product 5-(Piperazinocarbonyl)-5H-dibenzo-[b,f]azepine Hydrobromide in place of the product of step 2, and glutaric anhydride in place of succinic anhydride in the procedures of step 3. The residue (2B) was crystallized from ethyl acetate (10 mL) and petroleum ether (2 mL), placed in a freezer, and filtered to give the acid.

Step 3: 5-[4-(3-Carboxypropionyl)piperazinocarbonyl]-5H-dibenzo[b,f]azepine

This material was prepared using the procedures outlined in step 2B of Preparatory Example 4, except using succinic anhydride in place of glutaric anhydride. A sample recrystallized from dichloromethane/ethyl acetate (1:1) gave pure material.

Step 4: 5-[4-(4-Succinimidoxycarbonylbutyryl)piperazinocarbonyl]-5H-dibenzo[b,f]azepine

This material was prepared using the procedure outlined in Preparatory Example 2, step 4, except using 5-[4-(carboxybutyryl)piperazinocarbonyl]-5H-dibenzo[b,f]azepine in place of the N-[3-(3-carboxypropionamido)propyl]carbamazepine to give 4.6 g (100% yield). The material (3.0 g) was chromatographed using silica gel. Analytical calculated for $C_{28}H_{28}N_4O_6$: C, 65.09; H, 5.47; N, 10.85. Found: C, 64.87; H, 5.99; N, 10.62.

Preparatory Example 6

5-[4-(3-Succinimidoxycarbonylpropionyl)piperazinocarbonyl]-5H-dibenzo[b,f]azepine

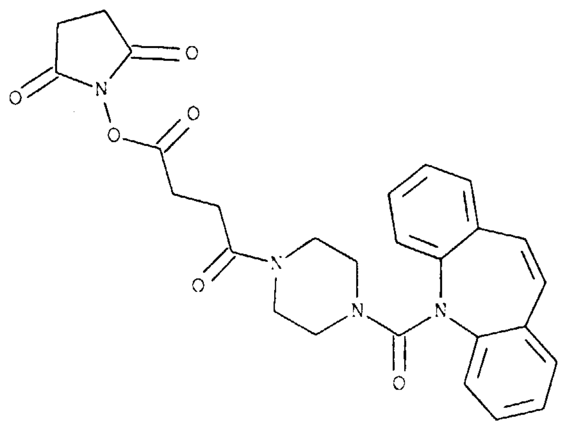

This material was prepared using the procedure outlined in step 4, Preparatory Example 2, except using 5-[4-(3-carboxypropionyl)piperazinocarbonyl]-5H-dibenzo[b,f]azepine in place of the N-[3-(3-carboxypropionamido)propyl] carbamazepine. A sample was recrystallized from dichloromethane (35 mL) and ethyl acetate (8 mL) to give material melting at 135-140°C. Analytical calculated for $C_{27}H_{26}N_4O_6$: C, 64.33; H, 5.22; N, 11.15. Found: C, 62.46; H, 5.29; N, 10.82.

Preparatory Example 7

N-(4-Succinimidoxycarbonylbutyl)carbamazepine

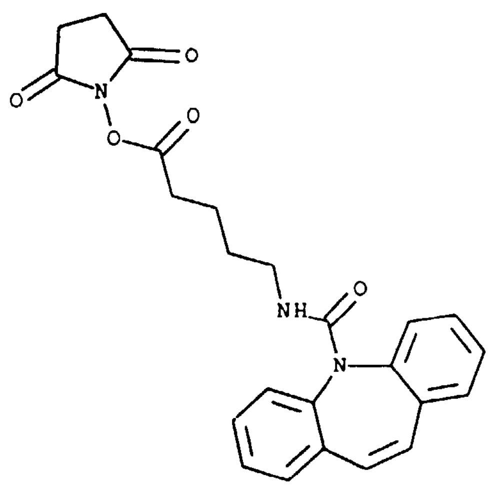

Step 1: N-(4-Methoxycarbonylbutyl)carbamazepine

To a mixture of sodium hydride (6.0 g, 0.2 mole, 80%) and carbamazepine (40.0 g, 0.17 mole) in tetrahydrofuran (400 mL) was added over 30 minutes methyl 5-bromovalerate (39.0 g, 0.19 mole) in tetrahydrofuran (100 mL). The mixture was stirred at ambient temperature for 3 days and then poured into ice containing concentrated hydrochloric acid (100 mL). The aqueous solution was extracted with dichloromethane (3 x 200 mL), and the combined organic solutions were washed with saturated sodium bicarbonate solution (200 mL), saturated sodium chloride solution (200 mL), then dried over anhydrous magnesium sulfate, filtered, and the solvent removed on a rotary evaporator in vacuo. To the residue was added ethyl ether (100 mL). The mixture was placed in the freezer (-16°C) and filtered to give a white solid.

Step 2: N-(4-Carboxybutyl)carbamazepine

N-(4-Methoxycarbonylbutyl)carbamazepine (6.3 g, 0.18 mole) was dissolved in p-dioxane (120 mL), water (25 mL), and concentrated hydrochloric acid (50 mL). The solution was refluxed for 2 hours and then stirred to ambient temperature. To this mixture was added saturated sodium chloride solution (100 mL) and the mixture was extracted with dichloromethane (3 x 300 mL). The combined organic solutions were washed with saturated sodium chloride solution (100 mL), dried over anhydrous magnesium sulfate, filtered, and the solvent removed. The residue was dissolved in dichloromethane (175 mL) and ethyl acetate (100 mL) was added. The mixture was placed in the freezer (-16°C) and then filtered.

Step 3: N-(4-Succinimidoxycarbonylbutyl)carbamazepine

This material was prepared using the procedure outlined in Preparatory Example 2, step 4, except using N-(4-carboxybutyl)carbamazepine in place of the N-[3-(3-carboxypropionamido)propyl]carbamazepine. Analytical calculated for $C_{24}H_{23}N_3O_5$: C, 66.50; H, 5.35; N, 9.69. Found: C, 65.82; H, 5.58; N, 9.37.

Preparatory Example 8

N-{4-[4-(3-Succinimidoxycarbonylpropionyl)piperazinocarbonyl]butyl}carbamazepine

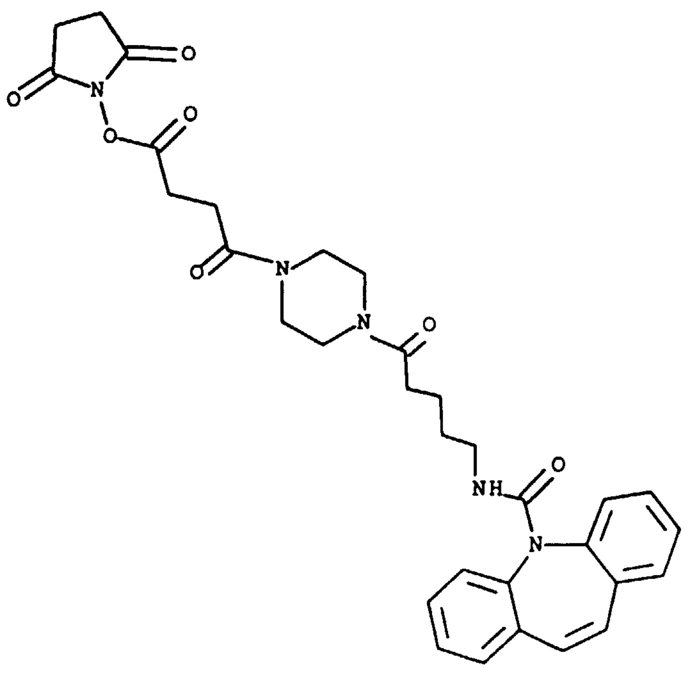

Step 1: N-[4-(4-Benzyloxycarbonylpiperazinocarbonyl)butyl]carbamazepine

A mixture of N-(4-carboxybutyl)carbamazepine (3.4 g, 0.01 mole) and 1,1'-carbonyldiimidazole (2.1 g, 0.0125 mole) in tetrahydrofuran (100 mL) was stirred at ambient temperature for 30 minutes. To this mixture was added at room temperature over 30 minutes benzyl 1-piperazinecarboxylate (2.75 g, 0.0125 mole) in tetrahydrofuran (100 mL). After 20 hours, dichloromethane (300 mL) was added, and the organic solution was washed with 5% hydrochloric acid solution (3 x 100 mL), washed with saturated sodium carbonate solution (100 mL), washed with saturated sodium chloride solution (100 mL), dried over anhydrous magnesium sulfate, filtered, and the solvent removed on a rotary evaporator in vacuo. The material was used directly in the next step.

Step 2A: N-(4-Piperazinocarbonylbutyl)carbamazepine Hydrobromide

Step 2B: N-{4-[4-(3-Carboxypropionyl)piperazinocarbonyl]butyl}carbamazepine

These materials were prepared using the procedures outlined in steps 2 and 3 of Preparatory Example 2, except starting with N-[4-(4-benzoyloxycarbonylpiperazinocarbonyl)butyl]carbamazepine in place of the N-[3-(benzoyloxycarbonylamino)propyl]carbamazepine in step 2A, and then using the product from step 2A of Preparatory Example 8 in place of the product of step 2 of Preparatory Example 2 in step 2B of Preparatory Example 8 to give the acid.

Step 3: N-{4-[4-(3-Succinimidoxycarbonylpropionyl)piperazinocarhonyl]butyl}carbamazepine

This material was prepared using the procedure outlined in Preparatory Example 2, step 4, except using N-{4-(4-(3-carboxypropionyl)piperazinocarbonyl]butyl}carbamazepine in place of the N-[3-(3-carboxropionamido)propyl]carbamazepine. A sample was chromatographed using silica gel to give analytically pure material. Analytical calculated for $C_{32}H_{35}N_5O_7$: C, 63.88; H, 5.86; N, 11.64. Found: C, 63.13; H, 6.02; N, 11.06.

Preparatory Example 9

N-{4-[3-(4-Succinimidoxycarbonylbutyramido)propylaminocarbonyl)butyl}carbamazepine

Step 1: N-[4-(3-Benzyloxycarbonylaminopropylaminocarbonyl)butyl]carbamazepine

This material was prepared using the procedure outlined in step 1 of Preparatory Example 8, except substituting N-benzyloxycarbonyl-1,3-propanediamine for the benzyl 1-piperazinocarboxylate. The residue was treated with ethyl ether (8 mL), acetone (4 mL), and petroleum ether (3 mL), placed in a freezer (-16°C), and filtered to give the product.

Step 2A: N-[4-(3-Aminopropylaminocarbonyl)butyl]carbamazepine Hydrobromide

Step 2B: N-{4-(3-(4-Carboxybutyramido)propylaminocarbonyl]butyl}carbamazepine

These materials were prepared using the procedures outlined in steps 2 and 3 of Preparatory Example 2, except substituting N-[4-(3-benzyloxycarbonylaminopropylaminocarbonyl)butyl]carbamazepine in place of the N-[3-(benzyloxycarbonylamino)propyl]carbamazepine in step 2, and N-[4-(3-aminopropylaminocarbonyl)butyl]carbamazepine hydrobromide for the N-(3-aminopropyl)carbamazepine hydrobromide and glutaric anhydride for the succinic anhydride in step 3, to give 2.6 g (44%) yield. The solid was recrystallized from methanol (4 mL) and ethyl acetate (15 mL) to give pure material.

Step 3: N-{4-[3-(4-Succinimidoxycarbonylbutyramido)propylaminocarbonyl]butyl}carbamazepine

This material was prepared using the procedures outlined in step 4 of Preparatory Example 2, except substituting N-{4-[3-(4-carboxybutyramido)propylaminocarbonyl]butyl}carbamazepine for the N-[3-(3-carboxypropionamido)propyl]carbamazepine. A sample was chromatographed on silica gel to give a white solid. Analytical calculated for $C_{32}H_{37}N_5O_7$: C, 63.67; H, 6.18; N, 11.60. Found: C, 61.74; H, 6.21; N, 10.77.

Example 10

Preparation of conjugate of N-(4-Succinimidoxycarbonyl-butyl)carbamazepine, and Amine-Enriched HRP (Horseradish Peroxidase) Conjugate (Label A)

Amine-enriched HRP was prepared as follows. Briefly, dry HRP (horseradish peroxidase) is dissolved in 0.1 M MES buffer, pH 5.5, to achieve a final concentration of 2.5 x $10^{-6}$ mol (100 mg) in 10 mL of buffer (MES = 2-(N-morpholino)ethane sulfonic acid). The protein concentration was determined by A403 measurement using the conversion factor A403 1 mg/mL = 2.24. The HRP solution was combined with 1.5 x $10^{-3}$ mol (275 mg) of L-lysine monohydrochloride dissolved in 10 mL of 0.1 M MES buffer at pH 5.5. A solution of freshly prepared 1-ethyl-3-(3-dimethylaminopropyl)carbodiimide hydrochloride (EDC, 5 x $10^{-4}$ mol, 960 mL) in MES buffer was added. The container was capped and mixed overnight at room temperature. The reaction was dialyzed against 0.02 M MOPS buffer at pH 7.0 (3 L at 10°C). The dialysis buffer was changed 3 times. MOPS = 3-(N-morpholino)propanesulfonic acid.

Prior to reaction, a sample of the amine-enriched HRP was exchanged from MOPS buffer into 0.1 M EPPS buffer,

pH 8.0, using Centricell Centrifugal Ultrafilter (30,000 nominal molecular weight limit). This sample was then diluted to 5.0 mL to produce a solution with a final concentration of 10.00 mg/mL (2.5 x $10^{-4}$ M). EPPS is N-(2-hydroxyethyl) piperazine-N'-3-propanesulfonic acid.

One mL of the amine-enriched HRP solution was added to a small vial (2.5 x $10^{-4}$ M). 500 mL of dimethylformamide, Aldrich 22,705-6, containing 10 mM 4'-hydroxyacetanilide (DMF 4'-HA) was added to the vial, vortexed, and placed in a 42°C water bath.

Meanwhile, the requisite carbamazepine analogue was dissolved in DMF 4'-HA to yield a 21.54 mg/mL solution (5.0 x $10^{-2}$ M). 500 mL of this solution was added to the HRP/DMF 4'-HA solution dropwise while vortex mixing. The molar ratio of the carbamazepine/HRP was 100/1.

Incubation was performed at 42°C for 1 hour with gentle shaking in a water bath. The sample was transferred to Spectrapor #2 dialysis tubing along with an additional 0.5 mL of DMF 4'-HA/0.1 M EPPS (1:1) used to rinse the reaction container.

The reaction was dialyzed as follows:

a) 1 L DMF 4'-HA/0.1 M EPPS, pH 8.0 (1:1), at 42°C for 1 hour
b) Dialysis condition a) was repeated once
c) 1.5 L 0.1 M EPPS, pH 8.0, containing 0.1% bovine serum albumin (BSA) at 5°C, 2 hours
d) 1.5 L 0.1 M EPPS, pH 8.0, at 5°C, overnight
e) 2.0 L 40 mM Tris-HCl, 150 mM NaCl, pH 7.5, for at least 8 hours
f) Dialysis condition e) was repeated once

Tris-HCl is tris(hydroxymethyl)aminomethane hydrochloride. Following dialysis, 0.02% merthiolate was added as a preservative, and the label was stored refrigerated.

Example 11

Conjugate of N-[6-(4-Succinimidoxycarbonylbutyramido)hexyl]carbamazepine and Amine-Enriched HRP (Label B)

Amine-enriched HRP was prepared as described in Preparatory Example 10. Prior to reaction, a sample of the amine-enriched HRP was exchanged from MOPS buffer into 0.1 M EPPS buffer, pH 8.0, using Centricell Centrifugal Ultrafilter (30,000 nominal molecular weight limit). This sample was then diluted to 3.0 mL to produce a solution with a final concentration of 8.36 mg/mL (2.08 x $10^{-4}$ M). EPPS is N-(2-hydroxyethyl)piperazine-N'-3-propanesulfonic acid.

To prepare Label B, one mL of amine-enriched HRP was added to a small vial (1.85 x $10^{-5}$ M). 500 mL of dimethylformamide, Aldrich 22,705-6, containing 10 mM 4'-hydroxyacetanilide (DMF 4'-HA) was added to the vial, vortexed, and placed in a 42°C water bath.

Meanwhile, the requisite carbamazepine drug analogue was dissolved in DMF 4'-HA to yield a 22.72 mg/mL solution (4.16 x $10^{-2}$ M). 500 mL of this solution was added to the HRP/DMF 4'-HA solution dropwise while vortex mixing. The molar ratio of the carbamazepine/HRP was 100/1.

Incubation was performed at 42°C for 1 hour with gentle shaking in a water bath. The sample was transferred to Spectrapor #2 dialysis tubing along with an additional 1 mL of DMF 4'-HA/0.1 M EPPS (1:1) used to rinse the reaction container.

The reaction was dialyzed and stored as described in Preparatory Example 10.

Example 12

Conjugate of N-{4-[4-(3-Succinimidoxycarbonylpropionyl)piperazinocarbonyl]butyl}carbamazepine and Amine-Enriched HRP (Label C); and

N-{4-[3-(4-Succinimidoxycarbonylbutyramido)propylaminocarbonyl]butyl}carbamazepine-Amine-Enriched HRP (Label D)

Amine-enriched HRP was prepared as described in Preparatory Example 10 and exchanged into 0.1 M EPPS buffer, pH 8.0, using Diaflo Ultrafilters YM30 filters (30,000 molecular weight cutoff) in Amicon Stirred Ultrafiltration Cells. This sample was then diluted to 3.18 mL to produce a solution with a final concentration of 10.00 mg/mL (2.5 x $10^{-4}$ M).

One mL of amine-enriched HRP was added to each of two small vials. 500 mL of dimethylformamide, Aldrich 22,705-6, was added to each vial, vortexed, and placed in a 42°C water bath for at least 15 minutes.

Meanwhile, Succinimidoxycarbonylpropionyl)piperazinocarbonyl]butyl}carbamazepine was dissolved in DMF to

yield a 30.05 mg/mL solution (2.5 x $10^{-2}$ M). To prepare Label C, 500 mL of this solution was added to the HRP/DMF solution dropwise while vortex mixing. Label D was prepared in the same manner, however, the drug analogue N-{4-[3-(3-Succinimidoxycarbonylbutyramido)propylaminocarbonyl]butyl}carbamazepine was dissolved in DMF to 30.15 mg/mL (2.5 x $10^{-2}$ M). The carbamazepine/HRP ratio for both labels is 100/1.

Incubation was performed at 42°C for 1 hour with gentle shaking in a water bath. The samples were transferred to Spectrapor #2 dialysis tubing along with an additional 1 mL of DMF/0.1 M EPPS (1:1) used to rinse the reaction container.

Each reaction mixture was dialyzed as follows:

a) 1 L DMF/0.1 M EPPS, pH 8.0 (1:1), at 42°C for 1 hour
b) Dialysis condition a) was repeated once
c) 2.0 L 0.1 M EPPS, pH 8.0, containing 0.1% bovine serum albumin (BSA) at 8°C, overnight
d) 2.0 L 0.02 M MOPS, pH 7.0, at 8°C, 8 hours
e) Dialysis condition d) was repeated twice, overnight and 8 hours

Following dialysis, 0.02% merthiolate was added as a preservative, and the label was stored refrigerated.

Example 13

Preparation of Carbamazepineanalogue-Amine-Enriched HRP Labels E, F, and G

Conjugate of N-[6-(4-Succinimidoxycarbonylbutyramido)hexyl]carbamazepine and amine enriched HRP (Label E);
Conjugate of N-{4-[4-(3-Succinimidoxycarbonylpropionyl)piperazinocarbonyl]butyl}carbamazepine and amine enriched HRP (F); and
Conjugate of N-{4-[3-(4-Succinimidoxycarbonylbutyramido)propylaminocarbonyl]butyl}carbamazepine and amine-enriched HRP (Label G)

Amine-enriched HRP was prepared as described in Preparatory Example 10 and exchanged into 0.1 M EPPS buffer, pH 8.0, to yield several mL of a 10.00 mg/mL solution (2.48 x $10^{-4}$ M). Three labels were prepared with 1 mL of amine-enriched HRP each. 500 mL DMSO, was added dropwise with vortex mixing to each sample. The samples were preincubated at room temperature for at least 20 minutes with shaking at 2400 rpm.

Meanwhile, N-[6-(4-Succinimidoxycarbonylbutyramido)hexyl]carbamazepine was dissolved in DMSO to yield a 27.08 mg/mL solution (2.48 x $10^{-2}$ M). Label E was made by adding 500 mL of this solution to one of the HRP/DMSO solutions prepared above. This was added dropwise while vortex mixing. The Labels F and G were prepared as described above, but the solutions were made as follows:

N-{4-[4-(3-Succinimidoxycarbonylpropionyl)piperazino-carbonyl]butyl}carbamazepine was dissolved to 29.81 mg/mL (2.48 x $10^{-2}$ M) and used to make Label F; N-{4-[3-(4-Succinimidoxycarbonylbutyramido)propylaminocarbonyl]butyl}carbamazepine was dissolved to 29.81 mg/mL (2.48 x $10^{-2}$ M) and used to make Label G. The molar ratio of all three labels was 100/1 carbamazepine to HRP.

Incubation was performed at room temperature for 4 hours with shaking at 2400 rpm. The samples were each transferred to Spectrapor #2 dialysis tubing along with an additional 1 mL of dialysate to rinse the reaction containers. The labels were dialyzed into 0.02 M MOPS buffer, pH 7.0, at 5-10°C. This dialysis condition was repeated 3 times with 2 to 3 L of buffer each time. Following dialysis, 0.02% merthiolate was added as a preservative, and the labels were stored refrigerated.

The immunoassay of this invention can be carried out in solution or on dry analytical elements.

The dry elements are generally considered to be more convenient. They can be single or multilayer or a combination of layers having zones within such layers. In general the elements can comprise a radiation transmissive support, one or more reagent layers, a particulate spreading layer preferably comprising beads upon which antibodies to the particular carbamazepine analyte are immobilized.

The layers can be coated using well known coating techniques in this art. For example, slide-extrusion hoppers of the type described in US-A-2,761,417 are often advantageous for simultaneous coating of a plurality of layers at least one of which is comprised of polymeric particles bearing immobilized antibody beads. More particularly, a multilayer element can be coated by directing a coating composition containing the beads through an extrusion slot of a slide extrusion hopper and simultaneously flowing a layer of a second coating composition, which, if desired, may also contain beads down a slide surface of the slide-extrusion hopper.

The particulate layer in which the antibodies are immobilized is porous. Materials for use in such layers are well known in the art of making dry analytical elements. A preferred particulate layer is a bead spreading layer (BSL). This layer can be easily constructed to have suitable porosity for use in the elements of the present invention to accommodate a test sample (e.g. 1 to 100 mL), diluted or undiluted. Preferably, the spreading layer is isotropically porous, which property is created by interconnected spaces between the particles comprising the zone. By isotropically porous is meant that the spreading layer uniformly spreads the applied fluid radially throughout the layer.

Useful particulate spreading layers, including bead spreading layers are disclosed in US-A-4,670,381; US-A-4,258,001 and US-A-4,430,436.

The particulate layer of the element is carried on a suitable support. Such a support can be any suitable dimensionally stable, and preferably, nonporous and transparent (i.e. radiation transmissive) material which transmits electromagnetic radiation of a wavelength between 200 and 900 nM. A support of choice for a particular element should be compatible with the intended mode of detection (reflection, transmission or fluorescence spectroscopy). Useful support materials include polystyrene, polyesters [e.g. poly(ethylene terephthalate)], polycarbonates, cellulose esters (e.g. cellulose acetate), and so forth.

The element can comprise one or more additional layers, e.g. separate or combined reagent/spreading layer and a gelatin/buffer layer containing other necessary additives, coupling enzymes, and so forth.

The gelatin/buffer layer or the reagent layer or the spreading layer of the element can contain the indicator composition comprising one or more reagents dispersed in one or more synthetic or natural binder materials, such as gelatin, or other naturally-occurring colloids, homopolymers and copolymers, such as poly(acrylamide), poly(vinyl pyrrolidone), poly(N-isopropylacrylamide), poly(acrylamide-co-N-vinyl-2-pyrrolidone) and similar copolymers.

Other optional layers, e.g. subbing layers, radiation-blocking layers, and so forth, can be included if desired. All layers of the element are in fluid contact with each other, meaning that fluids and reagents and uncomplexed reaction products in the fluids can pass between superposed regions of adjacent layers.

The layers of the element can contain a variety of other desirable but optional components, including surfactants, thickeners, buffers, hardeners, antioxidants, coupler solvents, and other materials known in the art. The amounts of these components are also within the skill of a worker in the art.

The elements can be used to determine low concentrations of carbamazepine in a liquid, such as a biological fluid (e.g., whole blood, serum, plasma, urine, spinal fluid, suspensions of human or animal tissue, feces, saliva, lymphatic fluid and the like). The carbamazepine can be determined at concentrations as low as $10^{-15}$ molar, and most generally at a concentration of from about $5.0 \times 10^{-6}$ to about $10^{-4}$ molar.

The assay can be carried out using any suitable label which can be attached to the defined carbamazepine analogues. Useful labels include radioactive tags, dyes, fluorescers, enzymes, enzyme substrates, enzyme inhibitors, allosteric effectors, cofactors, coenzymes and other known enzyme modulators. Enzymes, such as glucose oxidase, peroxidases such as horseradish peroxidase and amine-enriched horseradish peroxidase, alkaline phosphatase and galactosidase are preferred labels.

When an enzyme label is used, the substrate for the enzyme is present in the element or added thereto in the developing liquid. The substrate can be added to the element prior to or simultaneously with the liquid sample, or after completion of the binding reaction. It is within the skill of the ordinary worker in clinical chemistry to determine a suitable substrate for a given label. The substrate can be a material which is directly acted upon by the enzyme label, or a material that is involved in a series of reactions which involve enzymatic reaction of the label. For example, if the enzyme label is a peroxidase, the substrate is hydrogen peroxide. Using glucose oxidase as an example, the substrate glucose is generally present in the reagent layer or is added in the developing liquid to yield 0.01 moles/$m^2$, and preferably from 0.001 to 0.1 mole/$m^2$. A worker skilled in the art would know how to adjust the amount of a particular substrate for the amount of enzyme label used in the assay.

When certain labels are used, e.g. enzymes, cofactors, enzyme substrates or enzyme modulators, the reagent layer contains an indicator composition comprising one or more reagents which provide a detectable species as a result of reaction of the label. Preferably, the indicator composition is a colorimetric indicator composition which provides a colorimetrically detectable species as a result of enzymatic reaction of an enzyme-labeled ligand analog with a substrate.

The indicator composition can be a single compound which produces a detectable dye upon enzymatic reaction, or a combination of reagents which produce the dye. For example, when glucose is used as the substrate and glucose oxidase as the enzyme label, the colorimetric indicator composition can include a coupler and oxidizable compound which react to provide a dye. Alternatively, the composition can include a leuco dye and peroxidase or another suitable peroxidative compound which generates a detectable dye as a result of the formation of hydrogen peroxide produced when glucose oxidase converts glucose to gluconic acid. Useful leuco dyes are known in the art and include those, for example, described in US-A-4,089,747 and US-A-4,670,385. The particular amounts of the colorimetric indicator composition and its various components are within the skill of a worker in the art.

The immunoassay can be manual or automated. In general, the amount of a ligand in a liquid is determined by taking the element from a supply roll, chip packet or other source and physically contacting a finite area of the spreading layer with a sample of the liquid, e.g. 1 to 100 ml. The finite area which is contacted is generally no more than 100 $mm^2$.

The amount of ligand is determined by passing the element through a suitable apparatus for detecting the complexed ligand analog directly or the detectable species formed as a result of enzymatic reaction of an enzyme label and a substrate. For example, the species can be detected with suitable radiometric, fluorometric or spectrophotometric apparatus using generally known procedures. In an enzymatic reaction, the resulting product is determined by measuring, for example, the reflection or transmission density or fluorescence in the center of the finite area which was contacted with the test sample. The area which is measured is generally from 3 to 5 mm in diameter for competing assays. The amount of ligand in the liquid sample is inversely proportional to the amount of label measured in the center of the finite area. In a preferred embodiment a separate developer step is required in order to maximize separation of complexed ligand from uncomplexed ligand. Generally, label measurement is carried out after from 5 to 180 seconds after sample contact and spreading or application of the developing liquid.

The following examples demonstrate the utility of the novel labeled carbamazepine analogues in immunoassays and dry immunoassay elements.

Example 14

Performance of Carbamazepine-Amine-Enriched HRP Conjugates from Preparative Examples 10 to 13, Labels A to G.

Immobilized antibody beads were prepared by covalently attaching a carbamazepine monoclonal antibody (Kallestad prod. no. 330509) to poly[styrene-co-4-(2-chloroethylsulfonylmethyl)styrene] ( weight ratio 90/10) polymer beads of 0.47 mm average diameter.

The ability of immobilized carbamazepine antibody to bind carbamazepine-HRP labels was determined as indicated below:

The antibody beads were serially diluted with PBS containing 0.1% bovine serum albumin (BSA) to give antibody concentrations between 242 and 0.242 nM antibody binding sites. The bead dilutions were mixed with equal volumes of conjugate carbamazepine-HRP labels at 10 x $10^{-11}$ M. Following a 1 hour incubation, the beads were pelleted by centrifugation. A sample (100 mL) of the supernatant was mixed with 100 mL of substrate (o-phenylenediamine/$H_2O_2$). The rates of color development at 450 nM were compared with those of standards to calculate the amount of carbamazepine-HRP label remaining in solution. The amount of label bound to immobilized antibody at the highest antibody concentration of 121 nM binding sites is reported. The concentration of antibody binding sites where 50% of the label is bound is also reported.

| Preparative Example No. Analog | Label | Label | Preparation Solvent | % Label Bound at 121 nM Binding Sites | Ab Binding Sites at 50% Label |
|---|---|---|---|---|---|
| 7 | 10 | A | DMF | 18 | NA |
| 4 | 11 | B | DMF | 75 | 10.06 nM |
| 8 | 12 | C | DMF | 95 | 2.38 nM |
| 9 | 12 | D | DMF | 94 | 3.23 nM |
| 4 | 13 | E | DMSO | 85 | 5.55 nM |
| 8 | 13 | F | DMSO | 96 | 2.55 nM |
| 9 | 13 | G | DMSO | 97 | 2.47 nM |

Labels with longer linkers and those which contain piperazine are recognized better by the antibody. The percent label bound increases as linker length increases. Label E, made using DMSO, performed better than Label B. Preparations made using DMSO perform better than those made in DMF. DMSO allows for a more simple dialysis purification and results in improved or comparable performance.

Example 15

Use of a conjugate Carbamazepine-HRP Label Prepared with an Extended Linker in an Enzyme Immunoassay for Carbamazepine (CBZ).

This example demonstrates the preparation of an analytical element of this invention and its use in a competitive binding assay to detect the drug carbamazepine.

The immunoassay element was prepared to have the following structure using known technology described in the US-A-4,670,381; US-A-4,258,001 and US-A-4,430,436.

| | | Coverage (g/m²) |
|---|---|---|
| Spreading Layer | Immobilized Kallestad 330509 Antibody* | 0.2 |
| | Particles of poly[m-& p-vinyl toluene (64:36)-co-methacrylic acid] (98:2 weight ratio) (30 mm) | 130 |
| | 4,5-bis(4-dimethylaminophenyl)-2-(4-hydroxy-3,5-dimethoxyphenyl)imidazole leuco dye | 0.2 |
| | poly(methyl acrylate-co-sodium 2-acrylamido-2-methylpropanesulfonate-co-2-acetoacetoxyethyl methacrylate) (90:4:6 weight ratio) | 2.58 |
| | N-[tris(hydroxymethyl)methyl]-2-aminoethanesulfonic acid; (pH 7.0) | 0.219 |
| | dimethyl sulfoxide | 1.8 |
| | 5,5-dimethyl-1,3-cyclohexane dione | 0.50 |
| | Zonyl™ FSN nonionic surfactant (DuPont) | 0.054 |
| Gelatin Layer | hardened gelatin | 10 |
| | 4'-hydroxyacetanilide | 0.15 |
| | N-[tris(hydroxymethyl)methyl]-2-aminoethanesulfonic acid (pH 7.0) | 4.58 |
| | Triton™ X-100 nonionic surfactant (Rohm & Haas) | 0.02 |
| | Poly(ethylene terephtalate) Support | |

* The antibody was immobilized on poly[styrene-co-4-(2-chloroethylsulfonylmethyl)styrene] (weight ratio 90/10) polymer beads of about 0.47 mm average diameter by procedures as described by published EPA 88 307172.2.

A series of human serum based calibrators containing CBZ and a CBZ-HRP label (label C from Preparative Example 12) was prepared. The concentration of CBZ varied from 0.0 to 18.7 mg/mL. The conjugate CBZ-HRP label was added to give a final concentration of 2 nM.

The series of CBZ standards (10 mL aliquots) was spotted onto the spreading layers of a series of analytical elements. After 5 minutes incubation at 37°C, a wash solution (10 mL) comprising hydrogen peroxide (0.03%), sodium phosphate buffer (0.01 M, pH 6.8), 4'-hydroxyacetanilide (5 mM), diethylenetriaminepentaacetic acid (10 mM) and 0.1% 1-hexadecylpyridinium chloride was added to initiate dye formation. After about 1 minute, the reflection density ($D_r$) was measured at the center of the area at 680 nM at 37°C. The $D_r$ values were converted to $D_t$ by the Clapper-Williams transform. The change in $D_t$ over 60 seconds was calculated. The results are shown below:

| CBZ, mg/mL | Rate ($D_t$/min) |
|---|---|
| 0.0 | 0.1058 |
| 1.8 | 0.0466 |
| 5.0 | 0.0194 |
| 11.6 | 0.0086 |
| 18.7 | 0.0061 |

The results show that there is significant change in rates over the desired dynamic range. The therapeutic range is 8-12 mg/mL.

Example 16

Use of a conjugate Carbamazepine-HRP Label Prepared with an Extended Linker in an Enzyme Immunoassay for Carbamazepine (CBZ)

This example demonstrates the preparation of an analytical element of this invention and its use in a competitive binding assay to detect the drug carbamazepine.

The element was prepared using known technology to have the following structure:

| | | Coverage (g/m$^2$) |
|---|---|---|
| Spreading Layer | Immobilized 16.7.1 Antibody* | 0.2 |
| | Particles of poly[m-& p-vinyltoluene (64:36)-co-methacrylic acid](98:2 weight ratio)(30 mm) | 130 |
| | 4,5-bis(4-dimethylaminophenyl)-2-(4-hydroxy-3,5-dimethoxyphenyl) imidazole leuco dye | 0.2 |
| | poly(methyl acrylate-co-sodium 2-acrylamido-2-methyl-propanesulfonate-co-2-acetoacetoxyethyl methacrylate) (90:4:6 weight ratio) | 2.58 |
| | N-[tris(hydroxymethyl)methyl]-2-aminoethanesulfonic acid; (pH 7.0) | 0.219 |
| | dimethyl sulfoxide | 1.8 |
| | 5,5-dimethyl-1,3-cyclohexanedione | 0.50 |
| | Zonyl™ FSN nonionic surfactant (DuPont) | 0.054 |
| Gelatin Layer | hardened gelatin | 10 |
| | 4'-hydroxyacetanilide | 0.15 |
| | N-[tris(hydroxymethyl)methyl]-2-aminoethanesulfonic acid (pH 7.0) | 4.58 |
| | Triton™ X-100 nonionic surfactant (Rohm & Haas) Poly(ethylene terephthalate) Support | 0.02 |

* The antibody 16.7.1 was prepared and immobilized as described in EP-A-0 462 644 and EP-A-0 466 220 on poly(styrene-co-3-(p-vinylbenzylthio) propionic acid) (molar ratio 97.6/2.4; weight ratio 95/5) beads.

A series of human serum based calibrators containing CBZ and a CBZ-HRP label (label D from Preparative Example 12) was prepared. The concentration of CBZ varied from 0.0 to 18.7 mg/mL. The CBZ-HRP label was added to give a final concentration of 2 nM.

The series of CBZ standards (10 mL aliquots) was spotted onto the spreading layers of a series of analytical elements. After 5 minutes incubation at 37°C, a wash solution (10 mL) comprising hydrogen peroxide (0.03%), sodium phosphate buffer (0.01 M, pH 6.8), 4'-hydroxyacetanilide (5 mM), diethylenetriaminepenta-acetic acid (10 mM) and 0.1% 1-hexadecylpyridinium chloride was added to initiate dye formation. After about 1 minute, the reflection density ($D_r$) was measured at the center of the area at 680 nM at 37°C. The $D_r$ values were converted to $D_t$ by the Clapper-

Williams transform. The change in $D_t$ over 60 seconds was calculated. The results are shown below:

| CBZ, mg/mL | Rate ($D_t$/min) |
|---|---|
| 0.0 | 0.0896 |
| 1.8 | 0.0861 |
| 5.0 | 0.0515 |
| 11.6 | 0.0265 |
| 18.7 | 0.0129 |

The results show that there is significant change in rates over the desired dynamic range. The therapeutic range is 8-12 mg/mL.

## Claims

1. A labeled carbamazepine analogue comprising:

    (1) a label, of the type used in immunoassays, having an amine or sulfhydryl group;
    (2) a carbamazepine nucleus; and
    (3) a linking chain linking the carboxamide group of the carbamazepine nucleus to the label through a carbonyl group, said linking chain having 4 to 40 atoms selected from the group consisting of:

        (a) $C_2$ to $C_6$ alkylene groups; and
        (b) 5 to 7 membered heterocyclic ring groups selected from 1,4-piperazinylene; 2,5-dimethyl-1,4-piperazinylene; 1,3-imidazolidinylene, and 1,4-hexahydrodiazepinylene;
        each (a) and (b) group being joined into the linking chain through chemical groups selected from the group consisting of

            (a) esters, including thioesters

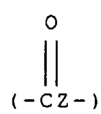

,

            where Z is O or S;
            (b) amides,

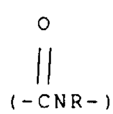

,

            wherein R represents hydrogen or $C_1$ to $C_6$ alkyl
            (c) hetero atoms selected from -O-, -S-, and -NR-; wherein each R independently represents hydrogen or $C_1$ to $C_6$ alkyl; and
            (d) carbonyl.

2. The labeled carbamazepine analogue according to claim 1 characterised in that it has the structure (I):

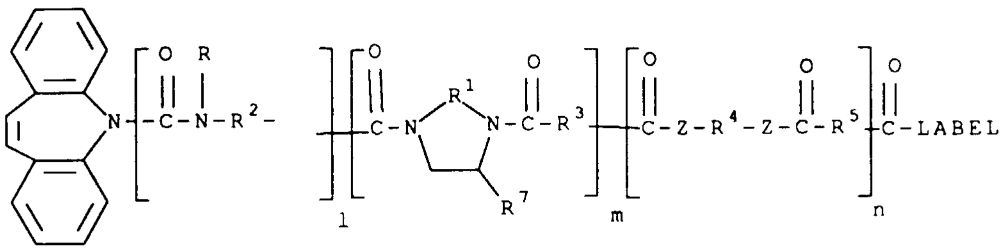

Structure I

wherein

R represents hydrogen or lower alkyl of 1 to 6 carbon atoms
$R^1$ is alkylene of 1 to 3 carbon atoms sufficient to form with $R^7$ a heterocylic group selected from 1,4-piperazinylene; 2,5-dimethyl-1,4-piperazinylene; 1,3-imidazolidinylene, and 1,4-hexahydrodiazepinylene;
$R^2$, $R^3$, $R^4$, and $R^5$ each independently represent alkylene groups of 2 to 10 carbon atoms, or
$R^7$ is hydrogen or methyl;
each Z independently represents -O-, -S-, or -NR- wherein R represents hydrogen or lower alkyl of 1 to 6 carbon atoms:
LABEL is an enzyme
l is 0, 1 or 2;
m is 1 or 2;
n is 0, 1, or 2; and

(i) the sum of carbon and hetero atoms in the linking chain, comprising the atoms included in $R^2$, $R^3$, $R^4$, $R^5$, $R_7$ and Z is 5 to 40, (ii) provided that the bracketed components l, m and n of structure I can appear therein in any order, and (iii) only one $R^2$, $R^3$, $R^4$, or $R^5$ group can be phenylene.

**3.** The labeled carbamazepine analogue according to claim 2 characterised in that:

$R^1$ represents ethylene thereby forming, with the atoms to which it is bonded, a 1,4-piperazinylene ring group;
$R^2$, $R^3$, $R^4$, and $R^5$ each independently, represent methylene, ethylene trimethylene, tetramethylene, pentylene, or hexylene; and
each Z independently represents -O-, -NH-,

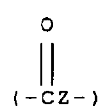

,

where Z is 0 or amide,

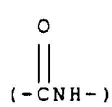

**4.** The labeled carbamazepine analogue according to claim 3 characterised in that $R^1$ is ethylene, $R^2$ is tetramethylene, $R^3$ is ethylene, propylene, or hexylene, $R^4$ and $R^5$ are independently ethylene, propylene, or hexylene, $R^7$ is hydrogen and Z is -O- or -NH-.

**5.** The labeled carbamazepine analogue according to any one of the preceding claims characterised in that the label is selected from horseradish peroxidase (HRP) or amine enriched horseradish peroxidase (AHRP).

**6.** The labeled carbamazepine analogue according to claim 5 characterised in that it is a conjugate of amine enriched horseradish peroxidase and a carbamazepine analogue selected from the group consisting of:

N-[6-(4-Succinimidoxycarbonylbutyramido)hexyl]carbamazepine;
N-{4-[4-(3-Succinimidoxycarbonylpropionyl)piperazinocarbonyl]butyl}carbamazepine; and
N-{4-[3-(4-Succinimidoxycarbonylbutyramido)propylaminocarbonyl]butyl}carbamazepine.

**7.** An immunoassay method for carbamazepine comprising:

A. contacting a liquid sample, containing carbamazepine, with a labeled carbamazepine analogue in the presence of antibodies for carbamazepine under conditions that promote the formation of carbamazepine/antibody immunocomplexes; and
B. determining the quantity of the drug in the liquid by measuring bound or unbound labeled drug analogue;
characterized in that the labeled carbamazepine analogue has a structure according to any one of claims 1-6.

**8.** A method according to claim 7 characterised in that it is carried out on an immunoassay element.

**9.** A method according to claim 8 characterised in that it is carried out on an immunoassay element containing the antibodies in one layer or zone and a separate layer or zone containing the labeled carbamazepine analogue.

**10.** A method according to claim 8 or claim 9 characterised in that it is carried out on an immunoassay element in which the antibodies are immobilized on beads in a particulate layer and the labeled analogue is in a coating directly above the layer containing the antibodies.

**11.** An immunoassay element having a layer, zone or coating of a labeled carbamazepine described in any one of claims 1-6.

**Patentansprüche**

**1.** Markiertes Carbamazepin-Analoges, umfassend:

(1) eine Markierung des bei Immunoassays verwendeten Typs mit einer Amin- oder Sulfhydrylgruppe;
(2) einen Carbamazepin-Kern; und
(3) eine verknüpfende Kette, die die Carboxamidgruppe des Carbamazepin-Kerns mit der Markierung über eine Carbonylgruppe verknüpft, wobei die verknüpfende Kette 4-40 Atome umfaßt, die aus folgender Gruppe ausgewählt sind:

(a) $C_2$-$C_6$-Alkylengruppen; und
(b) 5- bis 7-gliedrige heterocyclische Ringgruppen, ausgewählt unter 1,4-Piperazinylen; 2,5-Dimethyl-1,4-piperazinylen; 1,3-Imidazolidinylen; und 1,4-Hexahydrodiazepinylen;

wobei die einzelnen Gruppen (a) und (b) in die verknüpfende Kette über chemische Gruppen eingebunden sind, die aus folgender Gruppe ausgewählt sind:

(a) Ester, einschließlich Thioester

$$(-\overset{\overset{\displaystyle O}{\|}}{C}Z-),$$

worin Z die Bedeutung O oder S hat;
(b) Amide,

$$(-\overset{\overset{O}{\|}}{C}NR-),$$

worin R Wasserstoff oder einen $C_1$-$C_6$-Alkylrest bedeutet;
(c) Heteroatome, die unter -O-, -S- und -NR- ausgewählt sind, wobei die einzelnen Reste R unabhängig voneinander Wasserstoff oder $C_1$-$C_6$-Alkyl bedeuten; und
(d) Carbonyl.

**2.** Markiertes Carbamazepin-Analoges nach Anspruch 1, dadurch gekennzeichnet, daß es die folgende Strukturformel (I) aufweist:

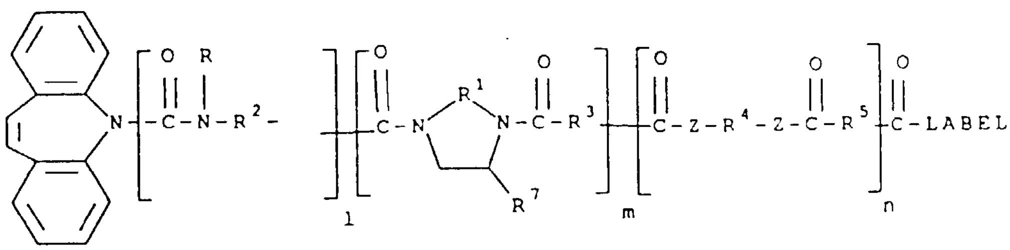

Strukturformel I

worin

R Wasserstoff oder einen niederen Alkylrest mit 1-6 Kohlenstoffatomen bedeutet,
$R^1$ Alkylen mit 1-3 Kohlenstoffatomen bedeutet, die zusammen mit $R^7$ zur Bildung einer heterocyclischen Gruppe ausreichen, die unter 1,4-Piperazinylen; 2-5-Dimethyl-1,4-piperazinylen; 1,3-Imidazolidinylen; und 1,4-Hexahydrodiazepinylen ausgewählt ist;
$R^2$, $R^3$, $R^4$ und $R^5$ jeweils unabhängig voneinander Alkylengruppen mit 2-10 Kohlenstoffatomen oder Phenylen bedeuten; $R^7$ Wasserstoff oder Methyl bedeutet;
die Reste Z unabhängig voneinander -O-, -S- oder -NR- bedeuten, wobei R Wasserstoff oder einen niederen Alkylrest mit 1-6 Kohlenstoffatomen bedeutet;
LABEL ein Enzym bedeutet,
l den Wert 0, 1 oder 2 hat;
m den Wert 1 oder 2 hat;
n den Wert 0, 1 oder 2 hat; und
(i) die Summe der Kohlenstoff- und Heteroatome in der verknüpfenden Kette, die die Atome in $R^2$, $R^3$, $R^4$, $R^5$, $R^7$ und Z umfaßt, 5-40 beträgt, (ii) mit der Maßgabe, daß die in Klammern gesetzten Komponenten 1, m und n der Strukturformel I in einer beliebigen Reihenfolge auftreten können und (iii) nur einer der Reste $R^2$, $R^3$, $R^4$ oder $R^5$ Phenylen bedeuten kann.

**3.** Markiertes Carbamazepin-Analoges nach Anspruch 2, dadurch gekennzeichnet, daß

$R^1$ Ethylen bedeutet, das zusammen mit den Atomen an die es gebunden ist, eine 1,4-Piperazinylen-Ringgruppe bildet;
$R^2$, $R^3$, $R^4$ und $R^5$ jeweils unabhängig voneinander Methylen, Ethylen, Trimethylen, Tetramethylen, Pentylen oder Hexylen bedeuten; und
die Reste Z jeweils unabhängig voneinander -O-, -NH-,

$$(-\overset{\overset{O}{\|}}{C}Z-),$$

worin Z die Bedeutung O oder Amid,

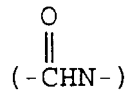

bedeutet.

4. Markiertes Carbamazepin-Analoges nach Anspruch 3, dadurch gekennzeichnet, daß $R^1$ Ethylen bedeutet, $R^2$ Tetramethylen bedeutet, $R^3$ Ethylen, Propylen oder Hexylen bedeutet, $R^4$ und $R^5$ unabhängig voneinander Ethylen, Propylen oder Hexylen bedeuten, $R^7$ Wasserstoff bedeutet und Z die Bedeutung -O- oder -NH- hat.

5. Markiertes Carbamazepin-Analoges nach einem der vorstehenden Ansprüche, dadurch gekennzeichnet, daß die Markierung unter Meerrettich-Peroxidase (HRP) oder mit Amin angereicherter Meerrettich-Peroxidase (AHRP) ausgewählt ist.

6. Markiertes Carbamazepin-Analoges nach Anspruch 5, dadurch gekennzeichnet, daß es sich um ein Konjugat von mit Amin angereicherter Meerrettich-Peroxidase und einem Carbamazepin-Analogen handelt, das aus folgender Gruppe ausgewählt ist:

N-[6-(4-Succinimidoxycarbonylbutyramid)-hexyl]-carbamazepin;
N-{4-[4-(3-Succinimidoxycarbonylpropionyl)-piperazinocarbonyl]-butyl}-carbamazepin; und N-{4-[3-(4-Succinimidoxycarbonylbutyramid)-propyl-amincarbonyl]-butyl}-carbamazepin.

7. Immunoassay-Verfahren für Carbamazepin, umfassend:

A. das Kontaktieren einer flüssigen Probe, die Carbamazepin enthält, mit einem markierten Carbamazepin-Analogen in Gegenwart von Antikörpern für Carbamazepin unter Bedingungen, die die Bildung von Carbamazepin/Antikörper-Immunokomplexen fördern; und
B. das Bestimmen der Menge des Arzneistoffs in der Flüssigkeit durch Messen von gebundenem und ungebundenem markiertem Arzneistoff-Analogen;

dadurch gekennzeichnet, daß das markierte Carbamazepin-Analoge eine Struktur nach einem der Ansprüche 1-6 aufweist.

8. Verfahren nach Anspruch 7, dadurch gekennzeichnet, daß es an einem Immunoassay-Element durchgeführt wird.

9. Verfahren nach Anspruch 8, dadurch gekennzeichnet, daß es an einem Immunoassay-Element durchgeführt wird, das in einer Schicht oder Zone, die Antikörper enthält, und in einer getrennten Schicht oder Zone, das markierte Carbamazepin-Analoge enthält, durchgeführt wird.

10. Verfahren nach Anspruch 8 oder 9, dadurch gekennzeichnet, daß es an einem Immunoassay-Element durchgeführt wird, bei dem die Antikörper an Kügelchen in einer teilchenförmigen Schicht immobilisiert sind und das markierte Analoge sich in einem Überzug direkt über der die Antikörper enthaltenden Schicht befindet.

11. Immunoassay-Element mit einer Schicht, einer Zone oder einem Überzug eines markierten Carbamazepins gemäß der Beschreibung in einem der Ansprüche 1-6.

## Revendications

1. Analogue marqué de la carbamazépine, comprenant :

(1) un marqueur, du type utilisé dans les analyses immunologiques, ayant un groupe amine ou sulfhydryle ;
(2) un noyau carbamazépine ; et
(3) une chaîne de liaison, qui relie le groupe carboxamide du noyau carbamazépine au marqueur par l'intermédiaire d'un groupe carbonyle, ladite chaîne de liaison ayant de 4 à 40 atomes choisis parmi l'ensemble

comprenant :

(a) les groupes alkylène en $C_2$-$C_6$ ; et
(b) les groupes à noyau hétérocyclique ayant de 5 à 7 chaînons choisis parmi le 1,4-pipérazinylène, le 2,5-diméthyl-1,4-pipérazinylène, le 1,3-imidazolidinylène et le 1,4-hexahydrodiazépinylène ;

chaque groupe (a) et (b) étant réuni à la chaîne de liaison par l'intermédiaire de groupes chimiques choisis parmi l'ensemble comprenant :

(a) les esters, y compris les thioesters

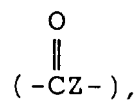

où Z est O ou S ;
(b) les amides,

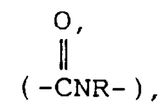

où R représente un atome d'hydrogène ou un groupe alkyle en $C_1$-$C_6$,
(c) les hétéroatomes choisis parmi -O-, -S- et -NR- ; où chaque radical R, indépendamment des autres, est un atome d'hydrogène ou un groupe alkyle en $C_1$-$C_6$ ; et
(d) le groupe carbonyle.

**2.** Analogue marqué de la carbamazépine selon la revendication 1, caractérisé en ce qu'il a la structure suivante :

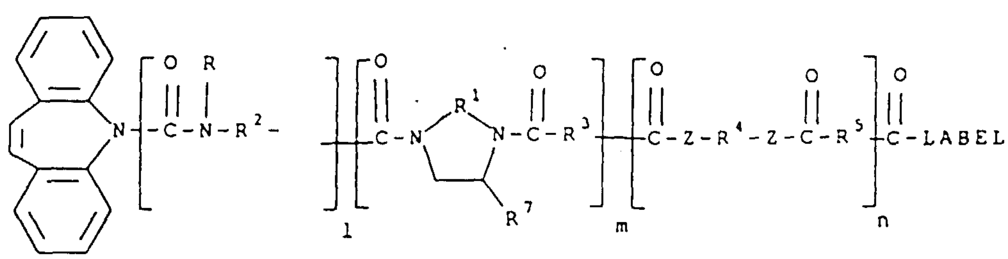

Structure I

Dans laquelle

R représente un atome d'hydrogène ou un groupe alkyle inférieur ayant de 1 à 6 atomes de carbone,
$R^1$ est un groupe alkylène ayant de 1 à 3 atomes de carbone, suffisant pour former avec $R^7$ est un groupe hétérocyclique choisi parmi le 1,4-pipérazinylène, le 2,5-diméthyl-1,4-pipérazinylène, le 1,3-imidazolidinylène et le 1,4-hexahydrodiazépinylène ;
$R^2$, $R^3$, R4 et $R^5$ sont chacun indépendamment des autres un groupe alkylène ayant de 2 à 10 atomes de carbone, ou phénylène ;
$R^7$ est un atome d'hydrogène ou le groupe méthyle ;
chaque Z, indépendamment des autres, représente -O-, -S- ou -NR-, où R représente un atome d'hydrogène ou un groupe alkyle inférieur ayant de 1 à 6 atomes de carbone ;
LABEL est une enzyme
l vaut 0, 1 ou 2 ;

m vaut 1 ou 2 ;
n vaut 0, 1 ou 2 ; et
(i) la somme des atomes de carbone et des hétéroatomes de la chaîne de liaison, y compris les atomes compris dans $R^2$, $R^3$, $R^4$, $R^5$, $R^7$ et Z, est de 5 à 40,
(ii) à la condition que les constituants 1, m et n présentés entre crochets dans la structure I puissent y apparaître dans un ordre quelconque, et
(iii) seulement l'un des groupes $R^2$, $R^3$, $R^4$ ou $R^5$ peut être le groupe phénylène.

**3.** Analogue marqué de la carbamazépine selon la revendication 2, caractérisé en ce que :

$R^1$ représente un éthylène, en formant ainsi, avec les atomes auxquels il est lié, un noyau cyclique 1,4-pipérazinylène ;
$R^2$, $R^3$, $R^4$ et $R^5$ représentent chacun indépendamment des autres un groupe méthylène, éthylène, triméthylène, tétraméthylène, pentylène ou hexylène, et
chaque radical Z, indépendamment des autres, représente -O-, -NH-,

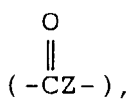

où Z est O ou un amide,

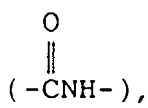

**4.** Analogue marqué de la carbamazépine selon la revendication 3, caractérisé en ce que $R^1$ est l'éthylène, $R^2$ est le tétraméthylène, $R^3$ est l'éthylène, le propylène ou l'hexylène, $R^4$ et $R^5$ sont chacun indépendamment de l'autre l'éthylène, le propylène ou l'hexylène, $R^7$ est un atome d'hydrogène et Z est -O- ou -NH-.

**5.** Analogue marqué de la carbamazépine selon l'une quelconque des revendications précédentes, caractérisé en ce que le marqueur est choisi parmi la peroxydase du raifort (HRP) ou la peroxydase du raifort enrichie en amine (AHRP).

**6.** Analogue marqué de la carbamazépine selon la revendication 5, caractérisé en ce qu'il s'agit d'un conjugué de la peroxydase du raifort enrichie en amine et d'un analogue de carbamazépine choisi parmi l'ensemble comprenant la N-[6-(4-succinimidoxycarbonylbutyramido)hexyl]-carbamazépine, la N-{4-[4-(3-succinimidoxycarbonylpropionyl)-pipérazinocarbonyl]butyl}carbamazépine et la N-{4-[3-(4-succinimidoxycarbonylbutyramido)-propylaminocarbonyl]butyl}-carbamazépine.

**7.** Méthode d'analyse immunologique pour doser la carbamazépine, qui consiste :

A. A mettre en contact un échantillon liquide, contenant de la carbamazépine, avec un analogue marqué de la carbamazépine en présence d'anticorps contre la carbamazépine dans des conditions qui favorisent la formation d'immunocomplexes carbamazépine/anticorps ; et
B. A déterminer la quantité du médicament dans le liquide en dosant l'analogue médicamenteux marqué fixé ou non fixé ; caractérisé en ce que l'analogue marqué de la carbamazépine a une structure selon l'une quelconque des revendications 1 à 6.

**8.** Procédé selon la revendication 7, caractérisé en ce qu'il est mis en oeuvre sur un élément d'analyse immunologique.

**9.** Procédé selon la revendication 8, caractérisé en ce qu'il est mis en oeuvre sur un élément d'analyse immunologique contenant les anticorps dans une couche ou dans une zone, et une couche ou une zone distincte contenant l'analogue marqué de la carbamazépine.

**10.** Procédé selon la revendication 8 ou 9, caractérisé en ce qu'il est mis en oeuvre sur un élément d'analyse immunologique dans lequel les anticorps sont immobilisés sur des perles se trouvant dans une couche particulaire, et l'analogue marqué se trouve dans un revêtement appliqué directement sur la couche contenant les anticorps.

**11.** Elément d'analyse immunologique comportant une couche, une zone ou un revêtement d'une carbamazépine marquée, décrite dans l'une quelconque des revendications 1 à 6.